(19) **Europäisches Patentamt**
**European Patent Office**
**Office européen des brevets**

(11) **EP 4 682 817 A1**

(12) **EUROPEAN PATENT APPLICATION**

(43) Date of publication:
**21.01.2026 Bulletin 2026/04**

(21) Application number: **24306206.4**

(22) Date of filing: **17.07.2024**

(51) International Patent Classification (IPC):
*G06T 5/50* (2006.01)

(52) Cooperative Patent Classification (CPC):
**G06T 5/50;** G06T 2207/20081; G06T 2207/20084;
G06T 2207/20221

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Dassault Systèmes**
**78140 Vélizy-Villacoublay (FR)**

(72) Inventors:
- **Mehr, Eloi**
  **Vélizy-Villacoublay (FR)**
- **Goldenberg, Louis, François**
  **Vélizy-Villacoublay (FR)**

(74) Representative: **Bandpay & Greuter**
**11 rue Christophe Colomb**
**75008 Paris (FR)**

(54) **IMAGING BASED ON A SET OF MEDICAL-IMAGING MODALITIES**

(57)   The disclosure notably relates to a computer-implemented method for machine-learning a function configured to take as input a plurality of aligned images of a same patient and each of a different modality among a predetermined set of medical-imaging modalities, and to calculate a fused image. The method comprises obtaining a dataset including, for each patient of a plurality of patients and for each modality of a respective at least part of the predetermined set, a respective image, the respective images for a patient being aligned; and training the function based on the dataset. This forms an improved solution for medical imaging.

EP 4 682 817 A1

## Description

### TECHNICAL FIELD

The disclosure relates to the field of computer programs and systems, and more specifically to methods, systems and data structures related to machine-learning and medical imaging.

### BACKGROUND

Medical imaging relates to imaging the interior of a body of a patient for clinical analysis and medical intervention, as well as visual representation of the patient's organs or tissues. The past decades have seen the advent of a multiplication of medical-imaging modalities, thanks to the diffusion of different imaging machines, sensors and/or technologies. Using medical-imaging modalities allows to visualize and/or process different aspects of tissue of a same patient, thereby capturing different pieces of medical information depending on the modality being visualized and/or processed. For example, a PET scan allows to visualize relatively clearly the physiological activity of a tumor, but this medical-imaging modality does not allow to capture accurately the contours of the tumor. On the contrary, on a CT scan, the physiological activity is less remarkable than on a PET scan, but the tumor's contours are visible in great detail. Combining the two modalities thus allows the practitioner to reach a higher level of medical information relevant for therapeutical use. However, as the number of possible medical-imaging modalities is high and the potential combinations are numerous depending on the patient's medical history or the medical situation/infrastructure, medical practitioners and/or processes suffer from a lack of ergonomics and/or standardization. Thus, navigating in this medical imaging environment is complicated.

Within this context, there is a need for an improved solution for medical imaging.

### SUMMARY

It is therefore provided a computer-implemented method (referred to as "machine-learning method") for machine-learning a function configured to take as input a plurality of aligned images of a same patient and each of a different modality among a predetermined set of medical-imaging modalities, and to calculate a fused image. The method comprises obtaining a dataset including, for each patient of a plurality of patients and for each modality of a respective at least part of the predetermined set, a respective image, the respective images for a patient being aligned. The method also comprises training the function based on the dataset.

The machine-learning method may further comprise one or more of the following:

- the function is configured to iteratively apply a fusion network to a pair of images so as to calculate the fused image, the pair of images comprising, at the first iteration, two images of the plurality of aligned images, and the pair of images comprising, at each subsequent iteration, one image of the plurality of aligned images and a result of applying the fusion network at the preceding iteration;
- the fusion network is identical at each iteration;
- the function is further configured to compute, from the fused image and for each modality of the predetermined set, a reconstructed image;
- the training comprises minimizing a loss which includes a sum, over the images of the dataset, of a reconstruction cost;
- the function is configured to take as input a variable number of images, including 2, and the loss further comprises a sum, over the images of the dataset, of a stability loss, the stability loss being represented, for each respective image of each respective patient, by a cost between (i) a first fused image calculated by applying the function with, as input, all the images of the respective patient included in the dataset, and (ii) a second fused image calculated by applying the function with, as input, the respective image and the first fused image;
- the loss further comprises an adversarial loss;
- the function is order-dependent with respect to the input plurality of images, the training comprising one or more applications of the function each with a respective input having a randomized order;
- the function is further configured to take as input, for a respective input image, a respective label representing the modality of the respective input image, and wherein optionally the training comprises one or more applications of the function each with a respective input including a respective fused image and a respective label representing a fusion nature of the respective fused image; and/or
- the predetermined set of medical-imaging modalities comprises one or more of the following modalities: Autorefraction, Angioscopy, Bone Densitometry (US), Biomagnetic Imaging, Bone Densitometry (X-Ray), Color Flow Doppler, Cinefluoroscopy, Colposcopy, Computed Radiography, Cystoscopy, Computed Tomography, Duplex Doppler, Digital Fluoroscopy, Diaphanography, Digital Microscopy, Digital Subtraction Angiography, Digital Radiography, Echocardiography, Electrocardiography, Cardiac Electrophysiology, Endoscopy, Fluorescein angiography, Fiducials, Fundoscopy, General Microscopy, Hard Copy, Hemodynamic Waveform, Intra-Oral Radiography, Intraocular Lens Data, Intravascular Optical Coherence Tomography, Intravascular Ultrasound, Keratometry, Lensometry, Laparoscopy, Laser Surface Scan, Magnetic Resonance Angiography, Mammography, Magnetic Resonance, MR T1 weighted, MR T2 weighted, MR Proton density weighted, MR Steady-state-free precession, MR Effective T2, MR Susceptibility-weighted, MR Short-tau inversion recovery, MR Fluid-attenuated inversion recovery, MR Double inversion recovery, MR Conventional diffusion weighted, MR Apparent diffusion coefficient, MR Diffusion tensor, MR Dynamic susceptibility contrast, MR Arterial spin contrast, MR Dynamic contrast enhanced, MR Blood-oxygen-level dependent imaging, MR Time-of-flight, MR Phase contrast, Magnetic Resonance Spectroscopy, Nuclear Medicine, Ophthalmic Axial Measurements, Optical Coherence Tomography (non- Ophthalmic), Ophthalmic Photography, Ophthalmic Mapping, Ophthalmic Refraction, Ophthalmic Tomography, Ophthalmic Visual Field, Optical Surface Scan, Other, Positron Emission Tomography (PET), Panoramic X-Ray, Respiratory Waveform, Radio Fluoroscopy, Radiographic Imaging (conventional film/screen), Radiotherapy Dose, Radiotherapy Image, Radiotherapy Plan, Radiotherapy Treatment Record, Radiotherapy Structure Set, Segmentation, Slide Microscopy, Stereometric Relationship, Single-Photon Emission Computed Tomography (SPECT), Automated Slide Stainer, Thermography, Utrasound, A-mode US, B-mode US, M-mode US, Visual Acuity, Videofluorography, X-Ray Angiography, External-Camera Photography.

It is further provided a method (referred to as "use method") of using a function having been machine-learnt by the above machine-learning method. The use method comprises inputting a plurality of aligned images of a same patient and each of a different modality among the predetermined set of medical-imaging modalities to the function. The use method also comprises, by the function, calculating a fused image with the input.

The use method may further comprise one or more of the following:

- the use method further comprises outputting and/or displaying the fused image;
- the use method further comprises reconstructing, for each of one or more modalities among the predetermined set of medical-imaging modalities, a respective reconstructed image;
- the one or more modalities among the predetermined set of medical-imaging modalities, for each of which the use method further comprises reconstructing a respective reconstructed image, include the modalities of the input;
- the use method further comprises outputting one or more reconstructed images; and/or
- the use method further comprises displaying one or more reconstructed images.

It is further provided a computer program comprising instructions for performing the machine-learning method and/or the use method.

It is further provided a function having been machine-learnt by the machine-learning method.

It is further provided a device comprising a data storage medium having recorded thereon the computer program and/or the function.

The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (e.g. the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIG. 1 shows a flowchart of an example of the machine-learning method;
- FIG. 2 shows a flowchart of an example of the use method;

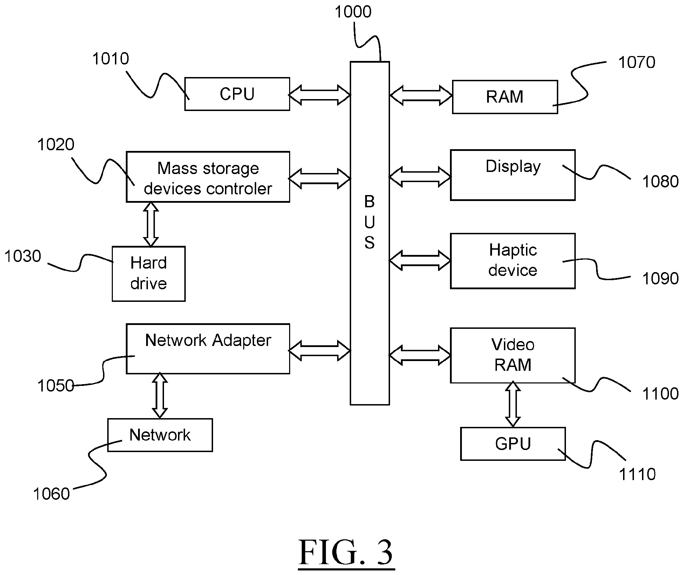

- FIG. 3 shows an example of the system; and
- FIG.s 4-12 illustrate the methods.

## DETAILED DESCRIPTION

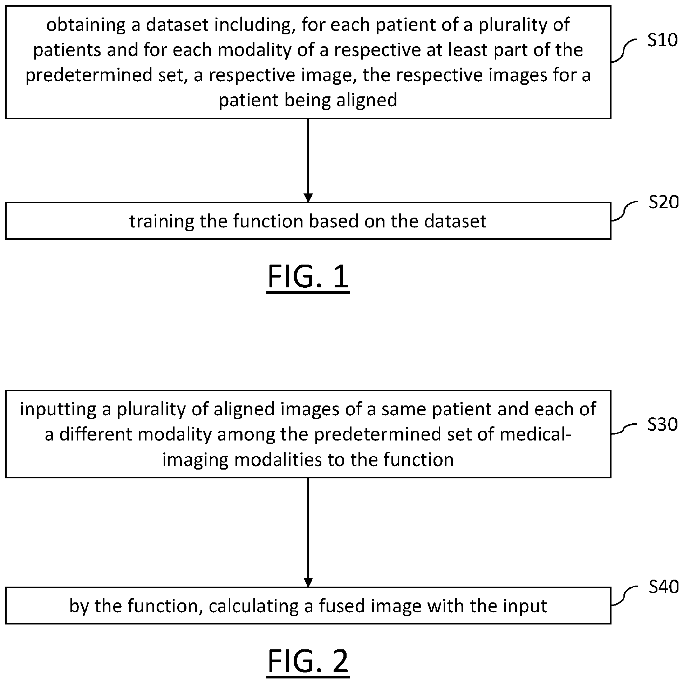

With reference to the flowchart of FIG. 1, it is proposed a computer-implemented method for machine-learning a function. The function is configured to take as input a plurality of images of a same patient which are aligned. Each image of the plurality is an image of a different modality among a predetermined set of medical-imaging modalities. Once machine-learnt, the function is configured to calculate a fused image from the input. The machine-learning method comprises obtaining S10 a dataset, and training S20 the function based on the dataset. The dataset includes training data relative to a plurality of patients. In particular, the dataset includes a respective training example (i.e., training pattern) for each patient of the plurality of patients. The training example for a respective patient includes a respective image for each modality of a respective at least part of the predetermined set of medical-imaging modalities. By "at least part of the predetermined set of medical-imaging modalities", it is meant a part of the predetermined set of medical-imaging modalities (i.e., one or more medical-imaging modalities forming a subset of the predetermined set), or, the whole predetermined set of medical-imaging modalities (i.e., all the medical-imaging modalities of the predetermined set). In the dataset, all the respective images of a same patient (i.e., in a same training example) are aligned (i.e., registered), if any (wherein, in examples, the dataset comprises, for one or more given patients, at least two respective images per given patient). Images are said to be "aligned" when they have the same dimensions (not necessarily the same resolution) and the same orientation, and they represent the same body portion of the patient in the same shape and size. In other words, the alignment ensures that any anatomical point is at the same position in all images. Thus, there is no deformation of the anatomy of the patient across different images that are aligned. For example, on an abdominal image, the organs can move depending on the patient's position during the acquisition, and aligning several abdominal images may comprise correcting such movements. In yet other words, the same pixel (or same pixel coordinates) of each image among several "aligned" images represents the same point in the patient's body.

The machine-learning method forms an improved solution for medical imaging.

In particular and with reference to the flowchart of FIG. 2, the machine-learning method allows to obtain a machine-learning function which, once trained (i.e., machine-learnt), may be involved in a use method. The use method comprises inputting S30 a plurality of images of a same patient to the function, and by the function, calculating S40 (and outputting) a fused image with the input. The plurality of images inputted at S30 are aligned. In addition, each image of the plurality inputted at S30 is an image of a different modality among the predetermined set of medical-imaging modalities.

The machine-learning function thus provides a way of merging medical images of different modalities in a joint representation, in the form of a fused image which aggregates information of multiple images in a single view. Thanks to the machine-learning nature of the proposed approach, the provided solution does not require ad-hoc fusion rule design and may be applied to a wide range of modalities. This has several medical applications.

The use method may for instance comprise displaying (a graphical representation of) the fused image, for example on a computer system display. The use method may further comprise a practitioner viewing the displayed fused image, and optionally making a medical assessment. Any medical assessment herein may comprise performing a diagnosis, a prognosis, or a determination or detection of any medical condition or of a value of a parameter relative to a medical condition, such as a segmentation and/or a measurement of a given body portion, and/or a determination of a medical treatment or of a medical treatment adjustment. Additionally or alternatively, the use method may comprise outputting the fused image, for example to a computer system or a processor, for example for automatic processing, such as automatic performance of a medical assessment. Thanks to the fused image representing in a single image a plurality of aligned images of a same patient and each of a different medical-imaging modality, the fused images allows an enhanced medical assessment.

For example, the predetermined set of medical-imaging modalities may comprise a high-resolution image modality, such as a Computed Tomography (CT) modality (also referred to as "CT scan" modality) or a Magnetic Resonance modality (MRI), and a lower-resolution image modality (i.e., having a resolution lower than the resolution of the high-resolution image modality) such as a Positron Emission Tomography (PET) modality, a Diffusion Tensor (DTI) modality, or an ultrasound modality. The dataset obtained at 10 may comprise training examples each including a respective image of the higher-resolution modality (e.g., CT scan) and a respective image of the lower-resolution modality (e.g., PET), both aligned. Optionally, for at least part of said training examples, the respective higher-resolution (e.g., CT scan) image and the respective lower-resolution (e.g., PET) image represent body tissue of a (same) patient containing tissue of a (same) tumor. The use method may in turn comprise inputting at S30 a plurality of images including one of the high-resolution modality (e.g., CT scan) and one of the lower-resolution modality (PET) which are aligned, e.g., and represent body tissue of a (same) patient containing tissue of a (same) tumor. In such a case, the function calculates at S40 a fused image of great use in oncology, as it achieves a merge of for example a CT scan, that images the patient anatomy with great detail, with for example a PET scan, that is often at a lower resolution but gives access to the physiological activity of tumors. The fused information can thus be used to couple both types of information in a single representation to benefit from the physiological information on a detailed view, that for example allows segmenting the contours, and measuring the size of the pathologic region. The proposed approach thus allows merging complementary medical images from different modalities into a single image.

By "medical-imaging modality", it is meant a type of imaging technique that utilizes a certain physical method to detect patient internal signals in order to observe either anatomical structures or physiological events. An image of a certain medical-imaging modality is thus the result of a transfer function of the biological, structural and physiological properties of the patient's tissues to an intensity space (generally $\mathbb{R}$ ) to reflect a desired property. Medical imaging modalities can differ by the physical mechanism they use, the physical sensor used to capture the image, the parameters of the sensor during the acquisition, the use of contrast agents, the delay between the injection of contrast agent and the acquisition, or the processing of the signal after the acquisition.

The predetermined set of medical-imaging modalities of the methods may comprise modalities involving acquisition with different physical mechanisms, modalities involving acquisition with different physical sensors, modalities involving acquisition with use of different contrast agents, modalities involving different delays between the injection of contrast agent and the acquisition, and/or modalities involving a different processing of the signal after the acquisition.

The predetermined set of medical-imaging modalities may (e.g., further) comprise one or more (e.g., any one, any combination, or all) of the following modalities: Autorefraction, Angioscopy, Bone Densitometry (US), Biomagnetic Imaging, Bone Densitometry (X-Ray), Color Flow Doppler, Cinefluoroscopy, Colposcopy, Computed Radiography, Cystoscopy, Computed Tomography (CT), Duplex Doppler, Digital Fluoroscopy, Diaphanography, Digital Microscopy, Digital Subtraction Angiography, Digital Radiography, Echocardiography, Electrocardiography, Cardiac Electrophysiology, Endoscopy, Fluorescein angiography, Fiducials, Fundoscopy, General Microscopy, Hard Copy, Hemodynamic Waveform, Intra-Oral Radiography, Intraocular Lens Data, Intravascular Optical Coherence Tomography, Intravascular Ultrasound, Keratometry, Lensometry, Laparoscopy, Laser Surface Scan, Magnetic Resonance Angiography, Mammography, Magnetic Resonance, MR T1 weighted, MR T2 weighted, MR Proton density weighted, MR Steady-state-free precession, MR Effective T2, MR Susceptibility-weighted, MR Short-tau inversion recovery, MR Fluid-attenuated inversion recovery, MR Double inversion recovery, MR Conventional diffusion weighted, MR Apparent diffusion coefficient, MR Diffusion tensor, MR Dynamic susceptibility contrast, MR Arterial spin contrast, MR Dynamic contrast enhanced, MR Blood-oxygen-level dependent imaging, MR Time-of-flight, MR Phase contrast, Magnetic Resonance Spectroscopy, Nuclear Medicine, Ophthalmic Axial Measurements, Optical Coherence Tomography (non- Ophthalmic), Ophthalmic Photography, Ophthalmic Mapping, Ophthalmic Refraction, Ophthalmic Tomography, Ophthalmic Visual Field, Optical Surface Scan, Other, Positron Emission Tomography (PET), Panoramic X-Ray, Respiratory Waveform, Radio Fluoroscopy, Radiographic Imaging (conventional film/screen), Radiotherapy Dose, Radiotherapy Image, Radiotherapy Plan, Radiotherapy Treatment Record, Radiotherapy Structure Set, Segmentation, Slide Microscopy, Stereometric Relationship, Single-Photon Emission Computed Tomography (SPECT), Automated Slide Stainer, Thermography, Utrasound, A-mode US, B-mode US, M-mode US, Visual Acuity, Videofluorography, X-Ray Angiography, External-Camera Photography.

The following table provides the standardized codification for these modalities:

| Code | Name |
|------|------|
| AR | Autorefraction |
| AS | Angioscopy |
| BDUS | Bone Densitometry (US) |
| BI | Biomagnetic Imaging |
| BMD | Bone Densitometry (X-Ray) |
| CD | Color Flow Doppler |
| CF | Cinefluoroscopy |
| CP | Colposcopy |
| CR | Computed Radiography |
| CS | Cystoscopy |
| CT | Computed Tomography |
| DD | Duplex Doppler |
| DF | Digital Fluoroscopy |
| DG | Diaphanography |
| DM | Digital Microscopy |
| DS | Digital Subtraction Angiography |
| DX | Digital Radiography |
| EC | Echocardiography |
| ECG | Electrocardiography |
| EPS | Cardiac Electrophysiology |

(continued)

| Code | Name |
| --- | --- |
| ES | Endoscopy |
| FA | Fluorescein angiography |
| FID | Fiducials |
| FS | Fundoscopy |
| GM | General Microscopy |
| HC | Hard Copy |
| HD | Hemodynamic Waveform |
| IO | Intra-Oral Radiography |
| IOL | Intraocular Lens Data |
| IVOCT | Intravascular Optical Coherence Tomography |
| IVUS | Intravascular Ultrasound |
| KER | Keratometry |
| LEN | Lensometry |
| LP | Laparoscopy |
| LS | Laser Surface Scan |
| MA | Magnetic Resonance Angiography |
| MG | Mammography |
| MR | Magnetic Resonance |
| MR-T1 | MR T1 weighted |
| MR-T2 | MR T2 weighted |
| MR-PD | MR Proton density weighted |
| MR-SSFP | MR Steady-state-free precession |
| MR-T2STAR | MR Effective T2 |
| MR-SWI | MR Susceptibility-weighted |
| MR-STIR | MR Short-tau inversion recovery |
| MR-FLAIR | MR Fluid-attenuated inversion recovery |
| MR-DIR | MR Double inversion recovery |
| MR-DWI | MR Conventional diffusion weighted |
| MR-ADC | MR Apparent diffusion coefficient |
| MR-DTI | MR Diffusion tensor |
| MR-DSC | MR Dynamic susceptibility contrast |
| MR-ASL | MR Arterial spin contrast |
| MR-DCE | MR Dynamic contrast enhanced |
| MR-BOLD | MR Blood-oxygen-level dependent imaging |
| MR-TOF | MR Time-of-flight |
| MR-PC-MRA | MR Phase contrast |
| MS | Magnetic Resonance Spectroscopy |
| NM | Nuclear Medicine |
| OAM | Ophthalmic Axial Measurements |

(continued)

| Code | Name |
| --- | --- |
| OCT | Optical Coherence Tomography (non-Ophthalmic) |
| OP | Ophthalmic Photography |
| OPM | Ophthalmic Mapping |
| OPR | Ophthalmic Refraction |
| OPT | Ophthalmic Tomography |
| OPV | Ophthalmic Visual Field |
| OSS | Optical Surface Scan |
| OT | Other |
| PT | Positron Emission Tomography (PET) |
| PX | Panoramic X-Ray |
| RESP | Respiratory Waveform |
| RF | Radio Fluoroscopy |
| RG | Radiographic Imaging (conventional film/screen) |
| RTDOSE | Radiotherapy Dose |
| RTIMAGE | Radiotherapy Image |
| RTPLAN | Radiotherapy Plan |
| RTRECORD | Radiotherapy Treatment Record |
| RTSTRUCT | Radiotherapy Structure Set |
| SEG | Segmentation |
| SM | Slide Microscopy |
| SMR | Stereometric Relationship |
| ST | Single-Photon Emission Computed Tomography (SPECT) |
| STAIN | Automated Slide Stainer |
| TG | Thermography |
| US | Utrasound |
| US-A | A-mode US |
| US-B | B-mode US |
| US-M | M-mode US |
| VA | Visual Acuity |
| VF | Videofluorography |
| XA | X-Ray Angiography |
| XC | External-Camera Photography |

The dataset may represent all modalities of the predetermined set of medical-imaging modalities. Thus, for each respective medical-imaging modality of the predetermined set, one or more training examples may include a respective image of the respective modality. In addition, the dataset obtained S10 may be such that all modalities of the predetermined set are interconnected. In other words, the graph defined as follows is a connected graph: each node of the graph corresponds to a respective modality of the predetermined set and each modality of the predetermined set has a respective node, and edges are defined between two nodes if, and only if, the two nodes correspond to a pair of modalities represented in a same training example (i.e., for at least one same patient, images of the two modalities are present in the dataset).

The dataset may comprise more than 100, 200 or 500 training examples (patients of which data are provided).

Additionally or alternatively, the predetermined set of medical-imaging modalities may comprise two or more modalities, for example more than 3, 5 or 10 modalities. Additionally or alternatively, the dataset may comprise, for each respective modality of the predetermined set of medical-imaging modalities, more than 100 or 200 training examples including an image of the respective modality. Additionally or alternatively, the dataset may comprise, for each respective pair of modalities of the predetermined set of medical-imaging modalities, more than 100 or 200 training examples including an image of each modality of the respective pair (i.e., connecting the two modalities of the pair within a training example).

The training at S20 is performed according to any machine-learning technique. The training S20 may comprise minimizing a loss over the dataset, by varying parameters and/or weights of the function. Such variable parameters and/or weights of the function are thus the trainable parameters and/or weights of the function. The minimizing may be performed in any manner, for example by a stochastic gradient descent.

The function may be further configured to compute, from the fused image and for each modality of the predetermined set, a reconstructed image. In other words, the function is structured and taught by the machine-learning method in way to be able to calculate, from the input plurality of images of S30, not only a fused image at S40, but also a synthetic image having the format and aspect of any respective one of the predetermined set of modalities.

In such a case, the use method may further comprise reconstructing a respective reconstructed image for each of one or more modalities among the predetermined set of medical-imaging modalities. The one or more modalities for which the method provides a respective reconstructed image may be defined in any manner, for example user-defined and/or predetermined (e.g., with a default behavior, e.g., which can be bypassed by the user), and/or for example include one or more (e.g., all) the modalities of the input provided at S30, and/or one or more other modalities (not provided at S30).

The function computes each such reconstructed image from the fused image. In other words, the function comprises a first component configured to take as input the plurality of aligned images of S30, and to calculate and output the fused image at S40. Also, the function comprises a second component (separate from the first component) configured to take as input (at least) the fused image (and optionally no other input, or, alternatively, another input such as one or more of the plurality of aligned images provided at S30), and to output a reconstructed image of any modality of the predetermined set of modalities. Optionally, the second component may comprise a respective subcomponent per respective reconstructed modality, each subcomponent being separate from each other subcomponent (i.e., each configured to take as input -at least- the fused image and to output the reconstructed image of the respective modality). The first component and the second component may each comprise distinct trainable parameters and/or weights. The trainable parameters and/or weights of the first component and the second component may all be varied and set within the same training S20. The subcomponents of the second component may each comprise distinct trainable parameters and/or weights. The trainable parameters and/or weights of the subcomponents may all be varied and set within the same training S20.

The first component, the second component, and/or each subcomponent of the second component may comprise or consist of any type of neural network, for example a respective convolutional neural network (CNN).

Thus, the function enables reconstructing and enriching the image of any modality included in S30 based on information contained in the other modality(ies) included in S30. Indeed, for a reconstructed image of a modality among those present at S30, the reconstruction is based on the fused image, such that the reconstructed image incorporates anatomical information captured by the other modalities present at S30. The function may thus be used to enhance each individual image provided at S30.

In addition, the function enables translating images of modalities included in S30 into images of other modalities (not included in S30), thereby enabling retrieving absent modalities for a given patient. Said translation is performed based on intermediary data, that is, the fused image calculated at S40. This improves accuracy of the translation, in the sense that a reconstructed image better represents the real anatomy of the patient.

Indeed, each such reconstructed image not only contains a translation of the information of one modality present at S30 into another modality (not present at S30), but as the translation takes into account the other images present at S30 (by fusing all the images provided at S30 and using the fused image as input of the translation), said reconstructed image also contains information of the other images.

Besides, the proposed approach addresses the scarcity of medical-imaging data, which may be an issue to perform effective machine-learning. Indeed, even if the dataset does not contain examples of how to translate modality A into modality C, if does contain examples of how to translate modality A into modality B and examples of how to translate modality B into modality C. By way of using a fused image as input of the translation, this is sufficient for the function to be trained to correctly translate modality A into modality C. In addition, even if the dataset contains examples of how to translate modality A into modality C, the function is trained to do such translation in a better way, as the training S20 can also benefit from the presence of examples of how to translate modality A into modality B and examples of how to translate modality B into modality C. In other words, using the fused image as an input to perform modality translation improves the machine-learning of modality translation, in a context of scarcity of training data as it relates to patient's data, and costly imaging techniques not systematically applied for every patient. Instead of separating available data in several small datasets each to train a specific function, the proposed approach allows for incomplete data to be relied upon so as to perform a single training based on one large dataset (thus richer, even if some training examples are incomplete).

In particular, the dataset obtained at S10 may in examples comprise, for at least part of the plurality of patients, incomplete data with respect to all of the predetermined set of medical-imaging modalities. In other words, for at least some patients, not all medical-imaging modalities are represented in the dataset, even though all medical-imaging modalities are represented overall in the dataset, when considering all patients. For example, the plurality of patients may comprise one or more first patients for which the training example includes, for each first patient, a respective image for each modality of a respective first subset of the predetermined set of medical-imaging modalities, and one or more second patients for which the training example includes, for each second patient, a respective image for each modality of a respective second subset of the predetermined set of medical-imaging modalities. The first subset and the second subset may have a non-empty intersection but be different, meaning that they both contain one or more medical-imaging modalities in common, but at least one of the first subset and second subset contains one or more medical-imaging modalities which are not contained in the other one of the first subset and second subset.

The use method may optionally comprise, additionally or alternatively to outputting and/or displaying the fused image, outputting one or more reconstructed images and/or displaying one or more reconstructed images. The use method may for instance comprise displaying (a graphical representation of) the one or more reconstructed images, for example on a computer system display. Optionally, the use method may comprise displaying several reconstructed images (each of a different modality) simultaneously on a single screen or on several screens. The use method may comprise user-selecting, at different times, several combinations of modalities, thus updating the displayed plurality of reconstructed images each time, based on the same input provided at S30. The practitioner is thus enabled to make assessment based on different modalities at will. The use method may further comprise a practitioner viewing the displayed one or more reconstructed images, and optionally making a medical assessment. Additionally or alternatively, the use method may comprise outputting the one or more reconstructed images, for example to a computer system or a processor, for example for automatic processing and automatic performance of a medical assessment.

The machine-learning function thus not only provides a way of merging medical images of different modalities in a joint representation, it also enables translating images from one or several modalities into an image of another modality. The machine-learning function in particular allows reconstructing images of original modalities, which is for example useful for translation from complementary images to a missing modality.

The proposed approach thus allows using the fused image to retrieve the original images, which enables for modality translation as it uses the available information from one or several images to reconstruct a missing modality. This gives access to a representation of the observed state of the patient in a modality that may be more practical for comparison or follow-up, without having to perform additional images.

Referring to the earlier-mentioned PET/CT example, the translation enables computing the CT from the PET scan, for example for attenuation correction purposes. Attenuation is a phenomenon that hinders the detection capabilities of PET scans, and it can be corrected using a CT-based density map to compensate the lost detections. Is this context, PET to CT translation allows reconstructing an approximate density map that is not used for diagnostic but to correct and enhance the capabilities of the PET scan alone, without having to perform a double acquisition.

The training S20 may comprise minimizing a loss which is a function of a reconstruction cost over (e.g., part or all) images present in the dataset. In other words, the training S20 minimizes globally reconstruction errors made by the function, when fusing together aligned images of different modalities representing a same patient tissue and then reconstructing individual images each of a respective modality from the fused image. The reconstruction cost measures a dissimilarity metric between an image of a given modality present in the dataset, and a reconstructed image of the same given modality outputted by the function, when inputted with the plurality of images of the initial training example. This enables an unsupervised and thus simple training. In addition, designing such a loss is simpler than designing ad-hoc fusion rules, and it achieves a high generalization capacity to various types of images. The designed loss function is not task-specific and can be applied to various image fusion tasks, such as visible/infrared, over/underexposed, far/near-focused, or PET/MRI). Moreover, relying on the fused image for translation in order to involve the available information from other modalities allows for reducing or preventing generation of artifacts in image translation (unlike approaches that based on a presence of semantics in the dataset, which is here lacking).

The aligned image(s) of at least one (e.g., each) training example obtained at S10 and/or the plurality of of aligned images of a same patient inputted at S30 may represent the same portion of a patient's body's interior, optionally at substantially the same time and/or without the body of the patient having substantially changed anatomically and physiologically during the interval. By "substantially the same time", it is meant that the images represent the body of the patient (e.g., were acquired on the patient) at times that are close enough so that the body of the patient has not substantially anatomically and physiologically changed in the interval, such that the images are comparable. For example, the images of a same patient at S10 and/or the images inputted at S30 may have been acquired on the patient all within one week, within two days, or within a single day. By no "substantial change in the patient's anatomy and physiology", it is meant that the patient's has not been affected by a major medical condition, that would prevent alignment of the images. Thus, the images of the patient can still be aligned. For example, no tumor has appeared in the interval separating two images. This concept is known from the field of medical imaging, where the technique of aligning images is known.

Depending on the predetermined set of medical-imaging modalities, each respective image of the dataset obtained at S10 may be 2D or respectively 3D. Correspondingly, each respective image inputted at S30 may be 2D or respectively 3D. If the dataset obtained at S10 comprises only 2D images, each respective image inputted at S30 may be 2D. In such a case, the fused image may be 2D. If the dataset obtained at S10 comprises only 3D images, each respective image inputted at S30 may be 3D. In such a case, the fused image may be 3D.

The fused image (that the function is trained to calculate at S20 and/or that is calculated at S40) may (e.g., systematically) be 2D or 3D, for example a 2D pixel image or a 3D voxel image.

Additionally or alternatively, the fused image may (e.g., systematically) be a one-channel image, that is, having a unique intensity value which is a (e.g., real or integer) number that can take value between a minimum value (e.g., 0) and a maximum value (e.g., 255). The fused image may for example comprise one, and only one, such intensity value for each pixel (if the fused image is a 2D pixel image), or for each voxel (if the fused image is a 3D voxel image). Thus, the function does not merely concatenate intensity values of the images inputted at S30, but it rather computes a (new) intensity value. The fused image may be a one-channel intensity map, and displaying the fused image may comprise performing an affine mapping (e.g., identity mapping) of the intensity domain onto a domain of grayscale values, and then computing and rendering a graphical representation of the result of the affine mapping. In an example, the function may directly output a 2D or 3D map of grayscale values (e.g., from 0 to 255).

Alternatively, the fused image may contain several channels if the input images contain several channels, or may contain only one channel but be displayed in color using a transformation of intensities to an RGB space (this technique is common in medical imaging).

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 2 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The function may be configured to take as input a variable number of images. Thus, the function may be applied and calculate at S40 a fused image whichever the number of images available at S30 for a given patient. The function may be configured to take as input a number of images equal to 2, and also (at the same time) a number of images higher than 2.

For example, the function may be configured to iteratively apply a fusion network to a pair of images so as to calculate the fused image. The pair of images comprises, at the first iteration, two images of the plurality of aligned images. The pair of images comprises, at each subsequent iteration, one image of the plurality of aligned images and a result of applying the fusion network at the preceding iteration (in other words, the output of the fusion network at the previous iteration). This allows the use of a canonical fusion network, configured to take a fixed number of images as input (2). Thus, the function does not depend on the size of the input plurality of images, as it operates the same way whichever the size, that is, pair-by-pair and iteratively fusing each pair. This facilitates the training and allows reaching an accurate function.

The fusion network applied at each iteration may be identical (i.e., the same fusion network is applied at each iteration). In other words, the fusion network forms a single component of the function, having its trainable parameters and/or weights, which is reused at each iteration of the iterative process. This again facilitates the training. As a result of a joint representation being learnt from several images of different modalities using a single fusion network, there is no constraint on the number of images or order of fusion.

The function may however be order-dependent with respect to the input plurality of images, meaning that the function takes as input a vector having coordinates each representing a respective image, and the function's architecture is such that the result is not the same depending on the ordering of the vector's coordinates. In such a case, the training S20 may comprise one or more applications of the function each with a respective input having a randomized order. In other words, the ordering of the plurality of images of each training example is randomized, for the purpose of the training. This teaches the function, which is initially (architecturally) order-dependent, to become order-independent, with respect to the input plurality of images. Thus, once trained, the function is substantially invariant to the ordering of the input plurality of images.

The function may be further configured to take as input, for a respective input image, a respective label representing the modality of the respective input image. This facilitates the training as it helps the function to learn focusing on other information than the modality of a given image.

In case the training S20 comprises (during the minimization of the loss) one or more applications of the function each with a respective input including a respective fused image, for example at subsequent iterations of application of a fusion network as discussed above, the value of the label for the fused image may be specific and distinct from the values of the labels representing the respective modalities of the predetermined set of medical-imaging modalities. This helps the function recognize when an input image is a fused image. Optionally, a unique label may be used during the training S20 to identify all fused images. The unique label thus represents a fusion nature of an input image, indistinctly of the modality or nature of the images from which the input image originates as a result of a fusion. This improves the training.

For each patient of the plurality of patients (in the dataset obtained at S120) and for at least one modality of the predetermined set, the respective image of the dataset (for said at least one modality) is a captured (i.e., acquired) image of the patient. In other words, the respective image is a physical/real image obtained from a real acquisition on the patient's body interior (contrary to a synthetic image).

Optionally, all images in the dataset are captured images.

Alternatively, the dataset may comprise synthetic images generated from such captured images; for example for patients for which captured images are initially missing. Optionally in such an alternative, synthetic images may be generated for each missing modality, or alternatively for only a part, thus still leaving missing modalities.

The method may comprise, during the training, applying a random mask to one or more images of the dataset, for example each image of the dataset, before the input of a respective image (to which a random mask is applied) to the function, (e.g., to the fusion network). Applying a random mask on a given image means that for a portion of the image, selected randomly (e.g., with predetermined and fixed shape and size but with a random location), the values in the image (e.g., intensity channel, e.g., at pixel or voxels of the image) are set to zero or null. Such a random mask helps the function become robust to missing data during the use method. And in case the dataset contains synthetic data, it helps the training S20 to avoid reproducing a mapping used for generating the synthetic data.

Examples of the method are now discussed referring to FIG.s 4-12.

The function may constitute an unsupervised deep learning model for merging images of different modalities into a joint image representation, which is able to reconstruct the original images from this representation. The function may be modality agnostic in its design, allowing variations in the considered modalities and order of fusion. Different examples of the methods discussed below may be developed on top of a base architecture, and differ in options implemented for the losses, the training means and some architectural choices.

The base architecture may consist in a unique fusion network, which iteratively merges images together to construct the joint representation, and reconstruction networks that retrieve the part of the fused image coming from a desired modality. This architecture allows fusing any number of images together into the joint representation without losing the essential information from each modality.

The model (i.e., function to be machine-learnt) may use registered multimodal data without any additional annotation or ground truth. If the data is not registered, this can be done during the preprocessing using existing methods, for example mutual information registration or DRMIME optimization as discussed below.

The proposed methods provide in examples a way of merging medical images of different modalities in a joint latent representation, and translating images from one or several modalities to another. Advantages of the solution in such examples are the followings:

- A joint representation is learnt from several images of different modalities using a single fusion network, there is no constraint on the number of images or order of fusion.

- Unlike classical image fusion technologies, the proposed solution does not require ad-hoc fusion rule design and is easily generalizable to a wide range of modalities.

- Unlike image fusion methods that only focus on the fused image quality, the proposed solution allows reconstructing the images of original modalities, which is useful for translation from complementary images to a missing modality.

- Unlike image translation methods that focus on style transfer or direct translation of images, the proposed solution provides a latent representation that aggregates the information of multiple images into a unique view.
- Unlike the CycleGAN model described in the paper by Zhu, J.-Y., Park, T., Isola, P., & Efros, A. A. (2020). Unpaired Image-to-Image Translation using Cycle-Consistent Adversarial Networks (arXiv:1703.10593), which is incorporated herein by reference, that can generate artifacts in image translation due to the lack of semantics in the data, the proposed solution relies on the fused image for translation in order to rely on the available information from other modalities.

*Data preparation*

In order to learn a joint latent representation of multimodal medical images, the machine-learning method may obtain at S10 a dataset of 2D or 3D medical images of different modalities. Images may be grouped by patient and aligned so that the anatomical regions visible in the different images match when images are superposed.

If no paired data is available, other technologies such as CycleGAN can optionally be used to generate synthetic data for means of training and testing the method.

If paired images corresponding to the same patient are available but not spatially aligned (i.e., registered), several technologies can be used for rigid registration of the images, e.g., mutual information registration (as described in paper by Xu, R., Chen, Y.-W., Tang, S.-Y., Morikawa, S., & Kurumi, Y. (2008). Parzen-window based normalized mutual information for medical image registration. IEICE Transactions on Information and Systems, 91(1), 132-144., which is incorporated herein by reference) or DRMIME optimization (as described in paper by Nan, A., Tennant, M., Rubin, U., & Ray, N. (2020). DRMIME: Differentiable Mutual Information and Matrix Exponential for Multi-Resolution Image Registration, arXiv:2001.09865, which is incorporated herein by reference).

*Neural network architecture*

The proposed approach relies on a core neural network model which can be extended by several options to address specific issues faced when trying to learn a joint representations and modality translation.

The core architecture may rely on a unique neural network $f_w$ parameterized by weights w. The network may take as input a stack of two images $(I_1, I_2)$ of different modalities $(M_1, M_2)$ and output a latent joint representation $I_F = f_w(I_1, I_2)$. The fusion network can be used iteratively to fuse an arbitrary number n of images coming from the same patient into a joint representation:

$$I_F = f_w(f_w(f_w(f_w(I_1, I_2), I_3), \dots), I_n)$$

In order to improve the robustness of the model, the machine-learning method may comprise training the fusion network $f_w$ so as to be invariant with respect to the fusion order. This may be done by randomly permuting the input images during the training, for example according to the following formula where $\sigma$ is a random permutation of $[\![1, n]\!]$ :

$$I_{F,\sigma} = f_w\big(f_w\big(f_w\big(f_w\big(I_{\sigma_1}, I_{\sigma_2}\big), I_{\sigma_3}\big), \ldots\big), I_{\sigma_n}\big)$$

The notation is dropped it in the remainder of the discussion. But the machine-learning method may be considered to apply the random permutation during training (unless explicitly mentioned).

Then the proposed approach adds multiple reconstruction networks $(g_i)_{1 \leq i \leq n}$ with weights $(w_i)_{1 \leq i \leq n}$ that take as input the joint latent representation $I_F$ and output the reconstructed image of the desired i-th modality:

$$\widehat{I_i} = g_i(I_F)$$

An option that is mathematically equivalent but can be advantageous from an implementation point of view is to use the same network $g$ for retrieving all modalities by adding a modality channel as input. This improves the feature extraction part of the network and is beneficial in some cases. The previous equation becomes $g(I_F, i)$ which is strictly equivalent, so the notation is dropped in the remainder of the discussion.

The machine-learning method may comprise training the model with a cycle-consistency loss, in order make reconstructed images match the original images. The loss may be written as follows, with $\mathcal{L}$ being a cost function to minimize (e.g., L1 or L2 cost function, or perceptual loss) and K being the number of patients in the dataset:

$$\min_{w, w_1, \ldots, w_n} \sum_{k=1}^{K} \sum_{i=1}^{n} \mathcal{L}(I_i, \hat{I}_i)$$

In case the dataset does not contain all the modalities for all the patients, an indicator function $\alpha$ may be introduced, such that $\alpha(k, i) = 1$ if the i-th modality is present for the k-th patient, and $\alpha(k, i) = 0$ otherwise. The loss function becomes:

$$\min_{w, w_1, \ldots, w_n} \sum_{k=1}^{K} \sum_{i=1}^{n} \alpha(k, i)\, \mathcal{L}(I_i, \hat{I}_i)$$

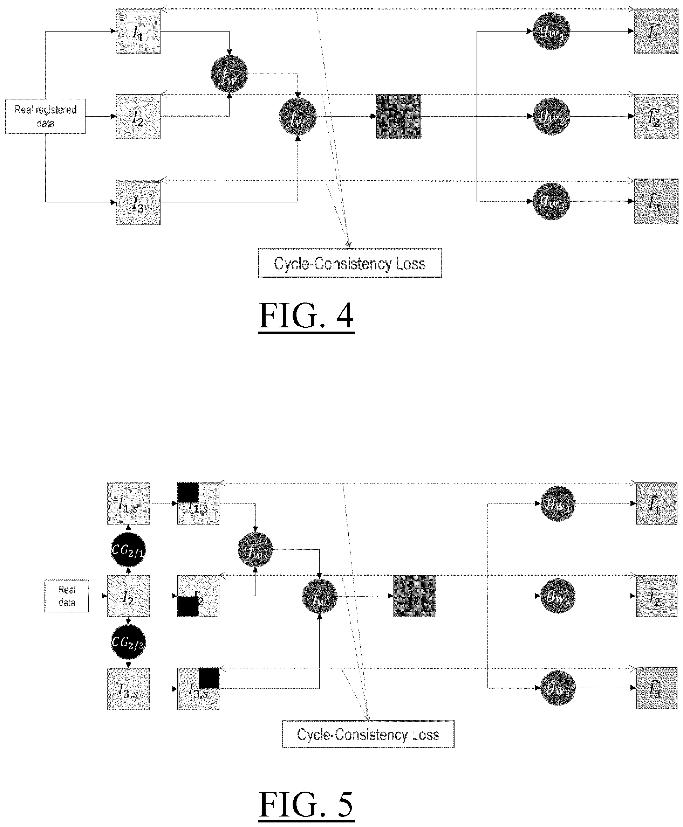

FIG. 4 shows an example of the architecture of the model, in the case of three images of different modalities being inputted.

Then, at inference time, the model can learn the joint representation $I_F$ using images $(I_1, \ldots, I_p)$ and infer the missing modalities $(I_{m_1}, \ldots, I_{m_r})$ using the retrieval networks.

$$I_F = f_w\big(f_w\big(f_w\big(f_w(I_1, I_2), I_3\big), \ldots\big), I_p\big)$$

$$I_{m_j} = g_{w_j}(I_F)$$

This reconstructed image benefits from the aggregated information of all present imaging modalities, and it can leverage on the existence of multiple observations. For example, the model may be used to reconstruct a CT scan using observations of an MRI for the structural information and a PET scan for the physiological information of tumors. The reconstructed CT scan may then be used for advanced visualization, segmentation and comparison by a practitioner.

*Model options*

Improving on this core architecture, the following proposes several options on loss design, model architecture, training strategies, and data management.

*Data management*

Data collection is an issue when working with medical images, and in this framework, one faces two main issues: having unregistered paired data, and not having paired data at all. As mentioned earlier, the machine-learning method may comprise preliminary registering unregistered data by other means and then treating said data using the same pipeline.

For unpaired data, an option of the base model is now discussed.

To face unpaired image data, that is when the dataset does not include images corresponding to the same patient or when this information is unavailable, the machine-learning method may comprise using a standard image translation method, such as CycleGAN, to create synthetic data in order to train the network.

An efficient training scheme to prevent the model from learning identical mappings to the CycleGAN and force it to extract more meaningful information from the joint representation, may be to use random masking. This way, missing information from each of the real or synthetic modality can be inferred in the joint representation and retrieved by reconstruction networks.

FIG. 5 illustrates this option, that is, the figure shows the architecture of the model with CycleGAN for the generation of synthetic data and random masking. The random masking option can be preserved for training even for cases of paired data in order to make the model more robust to missing information and to the inference of missing modality.

*Loss design and training strategies*

The reconstructed images may be oriented to match the original images during training by the cycle consistency loss. The loss may be imperfect to regularize the neural network and this could yield improvable generalization results.

The loss may further comprise an adversarial loss to account for that. Adversarial training can be used to improve the realism of reconstructed images. This is done with the introduction of discriminator networks $D_{u_i}$ parameterized by weights $u_i$ that predict the probability of their input being a true image of modality $M_i$.

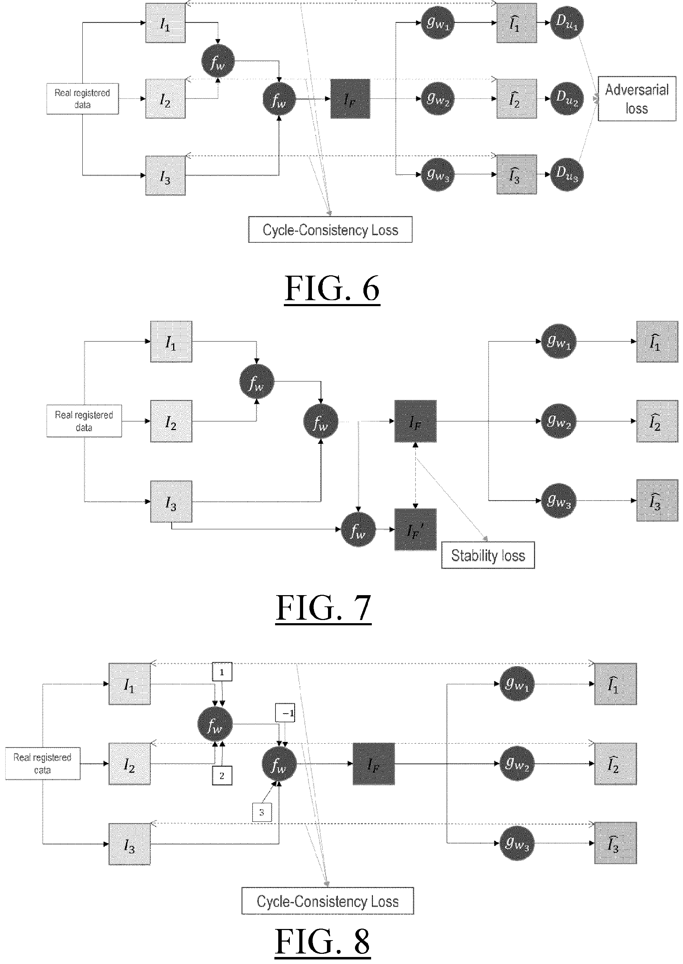

FIG. 6 illustrates this learning strategy, that is, with adversarial training.

The model's cost function becomes:

$$\min_{w, w_1, \ldots, w_n} \left[ \sum_{k=1}^{K} \sum_{i=1}^{n} \alpha(k, i) \, \mathcal{L}(I_i, \hat{I}_i) \right.$$

$$\left. + \max_{u_1, \ldots, u_n} \left[ \sum_{k=1}^{K} \sum_{i=1}^{n} \alpha(k, i) \log D_{u_i}(I_i) + \log\left(1 - D_{u_i}(\hat{I}_i)\right) \right] \right]$$

Referring to FIG. 7, another loss design consideration is that the joint representation may preferably not change when fusing in an image that it has already seen. For this purpose, the following model variation illustrated on the figure may be implemented. For each image $I_r$ that has already been fused in $I_F$ (in Figure 4 it is $I_3$), then merge it with $I_F$ and compare the result $I'_F = f_w(I_F, I_r)$ with $I_F$.

The loss function becomes:

$$\min_{w, w_1, \ldots, w_n} \sum_{k=1}^{K} \sum_{i=1}^{n} \alpha(k, i) \left[ \mathcal{L}(I_i, \hat{I}_i) + \mathcal{L}(I_F, f_w(I_F, I_i)) \right]$$

In other words, the loss further comprises a sum $\min_{w, w_1, \ldots, w_n} \sum_{k=1}^{K} \sum_{i=1}^{n} \alpha(k, i) \left[ \mathcal{L}(I_F, f_w(I_F, I_i)) \right]$,

over the images of the dataset, of a stability loss $\mathcal{L}$ $(I_F, f_w(I_F, I_i))$. The stability loss is represented, for each respective image of each respective patient, by a cost between (i) a first fused image $I_F$ calculated by applying the function with, as input, all the images of the respective patient included in the dataset, and (ii) a second fused image $f_w(I_F, I_i)$ calculated by applying the function with, as input, the respective image and the first fused image.

An additional option is to help the fusion network to identify the important information by using labels to indicate the type of modality used. This changes the function $f_w$ so that it takes the following arguments $f_w(I_i, i, I_j, j)$. From a network perspective, the label is given as a new channel uniformly containing the given label. Already fused images are labeled with -1 to make the distinction with original images.

FIG. 8 shows the architecture of the model with such labels for the fusion network.

*Implementation*

The fusion networks and reconstruction networks have been tested with a U-Net architecture (as presented in paper by Ronneberger, O., Fischer, P., & Brox, T. (2015). *U-Net: Convolutional Networks for Biomedical Image Segmentation,* arXiv:1505.04597, which is incorporated herein by reference) and a DenseNet architecture (as presented in papuer by Xu, H., Ma, J., Jiang, J., Guo, X., & Ling, H. (n.d.). *U2Fusion: A Unified Unsupervised Image Fusion Network.* IEEE TRANSACTIONS ON PATTERN ANALYSIS AND MACHINE INTELLIGENCE). The U-Net architecture is a type a convolutional network that has been developed for the purpose of handling medical image data, to capture high-level structure in the encoding part, and still retrieve fine detail with the skip connections at each level.

The U-Net architecture was found to perform better that DenseNets on the model.

*Applications*

The model was trained and tested on two datasets illustrating different situations. The following briefly describes both datasets and illustrates the results.

*Synthetic dataset*

The first tested dataset is a non-medical synthetic dataset containing ten thousand 2D images of spheres. The images were separated into three modalities according to Phong lighting model: ambient lighting, diffuse lighting and specular lighting. Eight thousand images were used for training, and a thousand was used for validation and training.

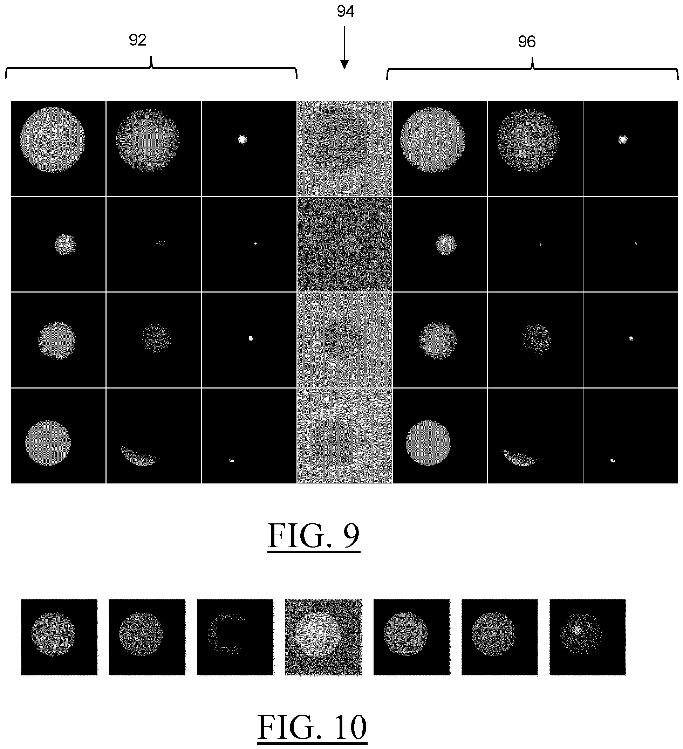

FIG. 9 illustrates the results obtained on four examples, the three left-most images 92 being the original images, the middle image 94 being the fused image, and the three right-most images 96 being the retrieved/reconstructed images.

FIG. 10 illustrates the interest of the multimodal translation on the same example, where the specular information was masked in the original images but retrievable using the ambient and diffuses components. The model efficiently performs the fusion of the available data and correctly translates it to the specular component.

*Medical dataset*

The second tested dataset is a medical dataset containing a thousand two hundred and fifty (1250) slices of brain MRI in both T1-weighted and T2-weighted modalities. They were separated into 900 images for training, 100 for validation and 250 for testing.

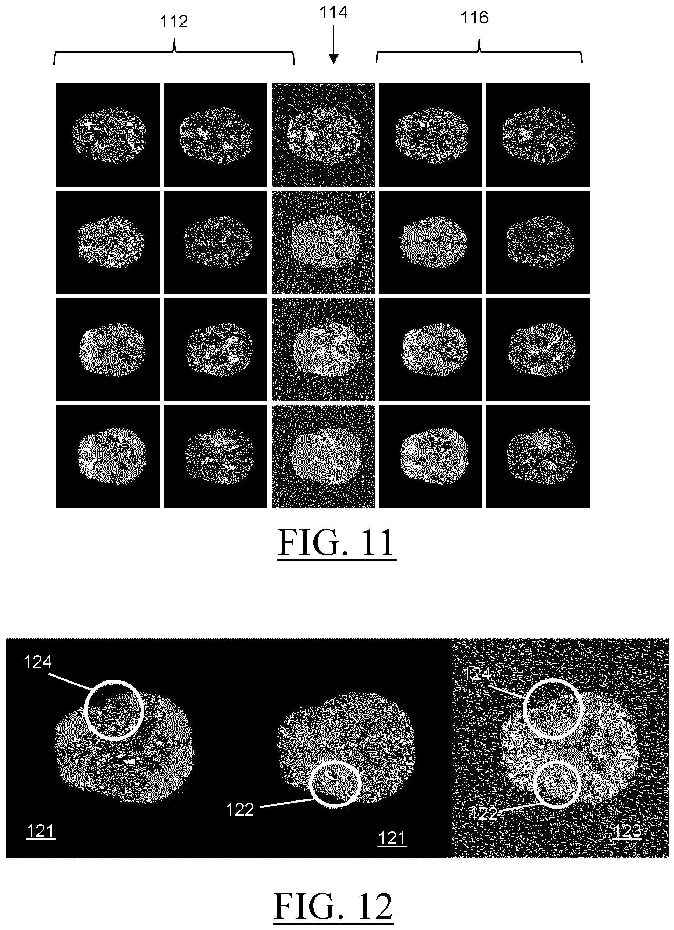

FIG. 11 illustrates the results obtained on four examples, the two left-most images 112 being the original images, the middle image 114 being the fused image, and the two right-most images 116 being the retrieved/reconstructed images. This illustrates the whole process of fusion and reconstruction of the original modalities that is done during training.

FIG. 12 illustrates how the fusion can aggregate information from both original images 121: the tumor is clearly visible on the T2 image and the detail is kept on the fused image 123 (circle 122), whereas the cortical details (circle 124) clearly come from the T1 image.

**Claims**

1. A computer-implemented method for machine-learning a function configured to take as input a plurality of aligned images of a same patient and each of a different modality among a predetermined set of medical-imaging modalities, and to calculate a fused image, the method comprising:

   • obtaining a dataset including, for each patient of a plurality of patients and for each modality of a respective at least part of the predetermined set, a respective image, the respective images for a patient being aligned; and
   • training the function based on the dataset.

2. The machine-learning method of claim 1, wherein the function is configured to iteratively apply a fusion network ($f_w$) to a pair of images so as to calculate the fused image ($I_F = f_w(f_w(f_w(f_w(I_1, I_2), I_3), ...), I_n))$), the pair of images comprising, at the first iteration, two images ($I_1, I_2$) of the plurality of aligned images, and the pair of images comprising, at each subsequent iteration, one image of the plurality of aligned images and a result of applying the fusion network at the preceding iteration.

3. The machine-learning method of claim 2, wherein the fusion network is identical at each iteration.

4. The machine-learning method of any one of claims 1 to 3, wherein the function is further configured to compute, from the fused image and for each modality (i) of the predetermined set, a reconstructed image ( $\widehat{I_i} = g_i(I_F)$ ).

5. The machine-learning method of claim 4, wherein the training comprises minimizing a loss which includes a sum (

$$\min_{w,w_1,\ldots,w_n} \sum_{k=1}^{K} \sum_{i=1}^{n} \alpha(k,i) \,[.]$$

), over the images of the dataset, of a reconstruction cost ( $\mathcal{L}$ $(I_i, \hat{I}_i)$).

6. The machine-learning method of claim 5, wherein:

   the function is configured to take as input a variable number of images, including 2, and

   the loss further comprises a sum ( $\min_{w,w_1,\ldots,w_n} \sum_{k=1}^{K} \sum_{i=1}^{n} \alpha(k,i) \,[.]$ ), over the images of the dataset, of a

   stability loss ( $\mathcal{L}$ $(I_F, f_w(I_F, I_i))$), the stability loss being represented, for each respective image of each respective patient, by a cost between (i) a first fused image ($I_F$) calculated by applying the function with, as input, all the images of the respective patient included in the dataset, and (ii) a second fused image ($f_w(I_F, I_i)$) calculated by applying the function with, as input, the respective image and the first fused image.

7. The machine-learning method of claim 5 or 6, wherein the loss further comprises an adversarial loss (

$$\max_{u_1,\ldots,u_n} \left[ \sum_{k=1}^{K} \sum_{i=1}^{n} \alpha(k,i) \log D_{u_i}(I_i) + \log\left(1 - D_{u_i}(\widehat{I_i})\right) \right]$$

).

8. The machine-learning method of any one of claims 1 to 7, wherein the function is order-dependent with respect to the input plurality of images, the training comprising one or more applications of the function each with a respective input having a randomized order.

9. The machine-learning method of any one of claims 1 to 8, wherein the function is further configured to take as input, for a respective input image, a respective label representing the modality of the respective input image, and wherein optionally the training comprises one or more applications of the function each with a respective input including a respective fused image and a respective label representing a fusion nature of the respective fused image.

10. The machine-learning method of any one of claims 1 to 9, wherein the predetermined set of medical-imaging modalities comprises one or more of the following modalities: Autorefraction, Angioscopy, Bone Densitometry (US), Biomagnetic Imaging, Bone Densitometry (X-Ray), Color Flow Doppler, Cinefluoroscopy, Colposcopy, Computed Radiography, Cystoscopy, Computed Tomography, Duplex Doppler, Digital Fluoroscopy, Diaphanography, Digital Microscopy, Digital Subtraction Angiography, Digital Radiography, Echocardiography, Electrocardiography, Cardiac Electrophysiology, Endoscopy, Fluorescein angiography, Fiducials, Fundoscopy, General Microscopy, Hard Copy, Hemodynamic Waveform, Intra-Oral Radiography, Intraocular Lens Data, Intravascular Optical Coherence Tomography, Intravascular Ultrasound, Keratometry, Lensometry, Laparoscopy, Laser Surface Scan, Magnetic Resonance Angiography, Mammography, Magnetic Resonance, MR T1 weighted, MR T2 weighted, MR Proton density weighted, MR Steady-state-free precession, MR Effective T2, MR Susceptibility-weighted, MR Short-tau inversion recovery, MR Fluid-attenuated inversion recovery, MR Double inversion recovery, MR Conventional diffusion weighted, MR Apparent diffusion coefficient, MR Diffusion tensor, MR Dynamic susceptibility contrast, MR Arterial spin contrast, MR Dynamic contrast enhanced, MR Blood-oxygen-level dependent imaging, MR Time-of-flight, MR Phase contrast, Magnetic Resonance Spectroscopy, Nuclear Medicine, Ophthalmic Axial Measurements, Optical Coherence Tomography (non- Ophthalmic), Ophthalmic Photography, Ophthalmic Mapping, Ophthalmic Refraction, Ophthalmic Tomography, Ophthalmic Visual Field, Optical Surface Scan, Other, Positron Emission Tomography (PET), Panoramic X-Ray, Respiratory Waveform, Radio Fluoroscopy, Radiographic Imaging (conventional film/screen), Radiotherapy Dose, Radiotherapy Image, Radiotherapy Plan, Radiotherapy Treatment Record, Radiotherapy Structure Set, Segmentation, Slide Microscopy, Stereometric Relationship, Single-Photon Emission Computed Tomography (SPECT), Automated Slide Stainer, Thermography, Utrasound, A-mode US, B-mode US, M-mode US, Visual Acuity, Videofluorography, X-Ray Angiography, External-Camera Photography.

11. A use method of a function having been machine-learnt by the machine-learning method of any one of claims 1 to 10, the use method comprising:

   • inputting a plurality of aligned images of a same patient and each of a different modality among the predetermined set of medical-imaging modalities to the function; and
   • by the function, calculating a fused image with the input.

12. The use method of claim 11, further comprising:

   • outputting and/or displaying the fused image, and/or
   • reconstructing, for each of one or more modalities among the predetermined set of medical-imaging modalities, optionally including the modalities of the input, a respective reconstructed image, and optionally outputting one or more reconstructed images and/or displaying one or more reconstructed images.

13. A data structure including:

   • a computer program comprising code instructions configured to cause a process to perform the machine-learning method of any one of claims 1 to 10 and/or the use method of claim 11 or 12; and/or
   • a function having been machine-learnt by the machine-learning method of any one of claims 1 to 10.

14. A data storage medium having stored thereon the data structure of claim 13.

15. A computer system comprising a processor coupled to a memory having stored thereon the data structure of claim 13.

obtaining a dataset including, for each patient of a plurality of patients and for each modality of a respective at least part of the predetermined set, a respective image, the respective images for a patient being aligned — S10

training the function based on the dataset — S20

## FIG. 1

inputting a plurality of aligned images of a same patient and each of a different modality among the predetermined set of medical-imaging modalities to the function — S30

by the function, calculating a fused image with the input — S40

## FIG. 2

1000

1010

CPU

1020

Mass storage
devices controler

1030

Hard
drive

Network Adapter

1050

Network

1060

B
U
S

1070

RAM

Display

1080

Haptic
device

1090

Video
RAM

1100

GPU

1110

FIG. 3

FIG. 4

FIG. 5

## FIG. 6

## FIG. 7

## FIG. 8

FIG. 9

FIG. 10

FIG. 11

FIG. 12

Europäisches
Patentamt

European
Patent Office

Office européen
des brevets

**EUROPEAN SEARCH REPORT**

**Application Number**

EP 24 30 6206

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIE YUCHAN ET AL: "Tri-Modal Medical Image Fusion and Denoising Based on BitonicX Filtering", IEEE TRANSACTIONS ON INSTRUMENTATION AND MEASUREMENT, vol. 72, 1 January 2023 (2023-01-01), pages 1-15, XP093231776, USA ISSN: 0018-9456, DOI: 10.1109/TIM.2023.3326255 Retrieved from the Internet: URL:https://ieeexplore.ieee.org/stampPDF/g etPDF.jsp?tp=&arnumber=10292538&ref=aHR0cH M6Ly9pZWVleHBsb3JlLmllZWUub3JnL2RvY3VtZW50 LzEwMjkyNTM4> | 1,2,8-15 | INV. G06T5/50 |
| A | * the whole document * ----- | 3-7 | |
| X | CN 116 205 847 A (GUANGDONG ZHUJIANG SWITCHGEAR CO LTD) 2 June 2023 (2023-06-02) | 1,2,8-15 | |
| A | * the whole document * ----- | 3-7 | **TECHNICAL FIELDS SEARCHED (IPC)** |
| X | DI WANG ET AL: "Unsupervised Misaligned Infrared and Visible Image Fusion via Cross-Modality Image Generation and Registration", ARXIV.ORG, CORNELL UNIVERSITY LIBRARY, 201 OLIN LIBRARY CORNELL UNIVERSITY ITHACA, NY 14853, 24 May 2022 (2022-05-24), XP091231521, | 1,4,5, 7-15 | G06T |
| A | * the whole document * ----- -/-- | 2,3,6 | |

The present search report has been drawn up for all claims

| Place of search | Date of completion of the search | Examiner |
|---|---|---|
| Munich | 5 December 2024 | Celik, Hasan |

EPO FORM 1503 03.82 (P04C01)

Europäisches
Patentamt

European
Patent Office

Office européen
des brevets

# EUROPEAN SEARCH REPORT

Application Number

EP 24 30 6206

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AZAM MUHAMMAD ADEEL ET AL: "A review on multimodal medical image fusion: Compendious analysis of medical modalities, multimodal databases, fusion techniques and quality metrics", COMPUTERS IN BIOLOGY AND MEDICINE, NEW YORK, NY, US, vol. 144, 3 February 2022 (2022-02-03), XP087012479, ISSN: 0010-4825, DOI: 10.1016/J.COMPBIOMED.2022.105253 [retrieved on 2022-02-03] * figure 5 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 5 December 2024 | Celik, Hasan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6206

This annex lists the patent family members relating to the patent documents cited in  the above-mentioned European search report.
The members are as contained in the European Patent Office EDP file on
The European Patent Office is in no way liable for these particulars which are merely given for the purpose of information.

05-12-2024

| Patent document cited in search report | Publication date | Patent family member(s) | Publication date |
|---|---|---|---|
| CN 116205847 A | 02-06-2023 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHU, J.-Y.** ; **PARK, T** ; **ISOLA, P.** ; **EFROS, A. A.** Unpaired Image-to-Image Translation using Cycle-Consistent Adversarial Networks. *arXiv:1703.10593*, 2020
- **XU, R** ; **CHEN, Y.-W** ; **TANG, S.-Y** ; **MORIKAWA, S.** ; **KURUMI, Y**. Parzen-window based normalized mutual information for medical image registration.. *IEICE Transactions on Information and Systems*, 2008, vol. 91 (1), 132-144

- **NAN, A.** ; **TENNANT, M.** ; **RUBIN, U.** ; **RAY, N.** DRMIME: Differentiable Mutual Information and Matrix Exponential for Multi-Resolution Image Registration. *arXiv:2001.09865*, 2020